# EUROPÄISCHE PATENTANMELDUNG

(11) **EP 2 325 173 A1**
(43) Veröffentlichungstag der Anmeldung: **25.05.2011**
(21) Anmeldenummer: 09176426.6
(22) Anmeldetag: 19.11.2009
(51) Int. Cl.: C07D 231/16

(54) **Verfahren zum Herstellen von 5-Fluor-1-alkyl-3-fluoralkyl-1H-pyrazol-4-carbonsäurechloriden**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein neues Verfahren zum Herstellen von 5-Fluor-1-alkyl-3-fluoralkyl-1H-pyrazol-4-carbonsäurechloriden, einer wertvollen Vorstufe für die Herstellung von Fungiziden.

## Beschreibung

Die vorliegende Erfindung betrifft ein neues Verfahren zum Herstellen von 5-Fluor-1-alkyl-3-fluoralkyl-1H-pyrazol-4-carbonsäurechloriden, einer wertvollen Vorstufe für die Herstellung von Fungiziden.

Pyrazolcarbonsäurechloride sind wichtige Bausteine zur Herstellung von Pflanzenschutzwirkstoffen. Pyrazolcarbonsäurechloride stellt man üblicherweise durch Umsetzung von Carbonsäuren mit einem Chlorierungsmittel dar. Ein Vorteil dieses Verfahrens beruht darauf, dass die entsprechenden Carbonsäuren einfach zugänglich und damit im technischen Maßstab verfügbar sind. Diese Vorrausetzung ist bei der Herstellung der substituierten Pyrazolcarbonsäurechloriden nicht gegeben, da die entsprechenden Carbonsäuren nicht leicht zugänglich sind.

Es wurde nun gefunden, dass man 5-Fluor-1-alkyl-3-fluoralkyl-1H-pyrazol-4-carbonsäurechloride der Formel (I) 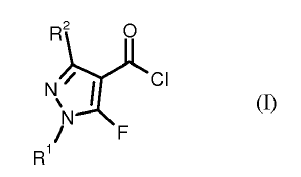 in welcher R¹ für C₁-C₆-Alkyl und R² für C₁-C₅-Fluoralkyl stehen, erhält, indem man
5-Chlor-1-alkyl-3-fluoralkyl-1H-pyrazol-4-carbaldehyd der Formel (II) 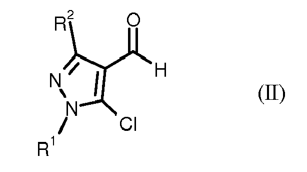 in welcher R und R² die oben angegebenen Bedeutungen haben,
zuerst (Schritt 1) mit einem Fluorierungsmittel der Formel (III)

M⁺ F⁻ (III)

in welcher M⁺ für Li⁺, Na⁺, K⁺, Cs⁺, Alk₄N⁺ steht (worin Alk für C₁-C₄-Alkyl steht),
und gegebenenfalls in Gegenwart eines Phasen-Transfer-Katalysators zu 5-Fluor-1-alkyl-3-fluoralkyl-1H-pyrazol-4-carbaldehyd der Formel (IV) 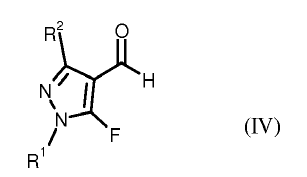 in welcher R¹ und R² die oben angegebenen Bedeutungen haben, umsetzt, und dann die Verbindungen der Formel (IV) durch Umsetzung mit einem Chlorierungsmittel (Schritt 2) in die Säurechloride der Formel (I) überführt.

Das erfindungsgemäße Verfahren kann durch das folgende Formelschema veranschaulicht werden:

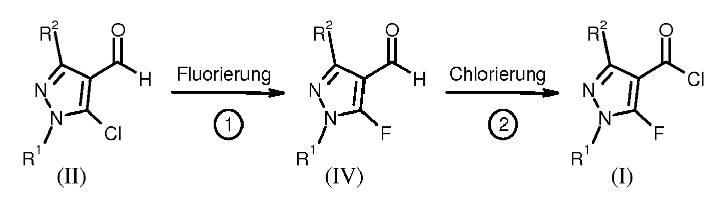

Die bei der Durchführung des erfindungsgemäßen Verfahrens als Ausgangsstoffe verwendeten 5-Chlor-1-alkyl-3-fluoralkyl-1H-pyrazol-4-carbaldehyde sind durch die Formel (II) allgemein definiert. Der Rest R¹ steht in dieser Formel (II) bevorzugt für Methyl, Ethyl, n-Propyl, Isopropyl, Butyl, Pentyl, besonders bevorzugt für Methyl. Der Rest R² steht für C₁-C₅-Fluoralkyl, worin Fluoralkyl eine Alkylgruppe mit 1 bis 5 Kohlenstoffatomen bedeutet, welche mit mindestens einem Fluoratom bis hin zur Perfluorierung substituiert ist. Wenn das Fluoralkyl nicht perfluoriert ist, können weitere Halogenatome wie Chlor und Brom, bevorzugt Chlor, als weitere Substituenten vorhanden sein. R² steht bevorzugt für CF₂H, CF₃, CF₂Cl, CCl₂F, C₂F₅, C₃F₇, besonders bevorzugt für CF₂H und CF₃. Ganz besonders bevorzugt setzt man 5-Chlor-1-methyl-3-difluormethyl-1H-pyrazol-4-carbaldehyd (II-1) als Ausgangsstoff ein.

5-Chlor-1-alkyl-3-polyfluorlkyl-1H-pyrazol-4-carbaldehyde der Formel (II) sind bekannt oder lassen sich nach bekannten Verfahren herstellen (vgl. J. Het. Chem. 1990, 27, 243, WO 2006/018725).

5-Chlor-1-alkyl-3-polyfluorlkyl-1H-pyrazol-4-carbaldehyde der Formel (II) lassen sich beispielsweise herstellen, indem man
(a) Ester der Formel (VI) 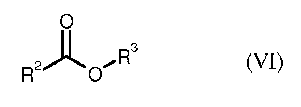 in welcher R² die oben angegebenen Bedeutungen hat und R³ für Methyl oder Ethyl steht,
   mit Ethylacetat in Gegenwart einer Base (z.B. Natriumhydrid) und in Gegenwart eines Verdünnungsmittels (z.B. Tetrahydrofuran) zu β-Ketoestern der Formel (VII) 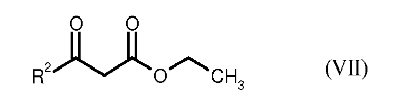 in welcher R² die oben angegebenen Bedeutungen hat, umsetzt, und diese
(b) mit Alkylhydrazinen der Formel (VIII) 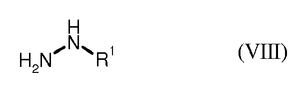 in welcher R¹ die oben angegebenen Bedeutungen hat,
   in Gegenwart eines Verdünnungsmittels (z.B. Toluol) zu 5-Hydroxy-pyrazolen der Formel (IX) 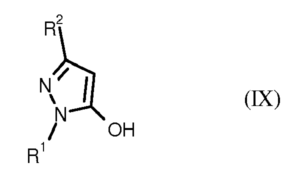 in welcher R¹ und R² die oben angegebenen Bedeutungen haben, umsetzt, und diese
(c) in einem letzten Schritt in Gegenwart mit einem Chlorierungsmittel (z.B. Phosphoroxychlorid) in Gegenwart eines Verdünnungsmittels (z.B. Toluol) und in Gegenwart von Dimethylformamid umsetzt.

Das weiterhin als Ausgangsstoff benötigten Fluoride wie z.B. Natrium- und Kaliumfluorid sind bekannte Synthesechemikalien.

### Schritt 1: Fluorierung

Verfahren zum Austausch von Chlor gegen Fluor (Halexprozesse) sind insbesondere für 5-Chloro-1,3-dilakyl-1H-pyrazol-4-carbosäurechloride bekannt (vgl. z.B. WO 2007/031212 und EP-A 0 776 889). Hierbei wird das Säurechlorid in das Säurefluorid umgewandelt und dadurch zusätzlich die Fluorierung beschleunigt. Falls die Aktivierungsgruppe jedoch der Aldehyd CHO anstelle des Säurechlorids COCI oder des Säurefluorids COF ist, liefert die Umsetzung mit Kaliumfluorid nur sehr niedrige Ausbeuten. Zum Beispiele wird 5-Fluor-1,3-dimethyl-1H-pyrazol-4-carbaldehyd durch Umsetzung von 1,3-Dimethyl-5-chlor-pyrazol-4-carbaldehyd mit Kaliumfluorid nur in 24 % Ausbeute (EP-A 0 776 889) erhalten. Als Ursache für die schlechte Ausbeute kommt auch die niedrige thermische Stabilität von Pyrazolaldehyden infrage (vgl. EP-A 1364 946). Es ist nicht vorauszusehen, ob die Fluorierung von 5-Chlor-1-alkyl-3-fluoralkyl-1H-pyrazol-4-carbaldehyde mit Metallfluoriden erfolgreich sein könnte.

Es ist als überraschend anzusehen, dass die Fluorierung von 5-Chlor-1-alkyl-3-fluoralkyl-1H-pyrazol-4-carbaldehyden mit Metallfluoriden selektiv und in hoher Ausbeute zum neuen 5-Fluor-1-alkyl-3-fluoralkyl-1H-pyrazol-4-carbaldehyd führt.

Die Reaktionstemperaturen können bei der Durchführung des erfindungsgemäßen Verfahrens in einem größeren Bereich variiert werden. Im Allgemeinen arbeitet man bei Temperaturen von 120°C bis 200°C, bevorzugt bei Temperaturen von 110°C bis 180°C.

Die Reaktionszeit kann in Abhängigkeit von der Reaktivität der Edukte bis zu 20 Stunden betragen, wobei der Abbruch der Reaktion bei vollständigem Umsatz auch schon früher erfolgen kann. Bevorzugt sind Reaktionszeiten von 3-10 Stunden.

Bei der Durchführung des erfindungsgemäßen Verfahrens setzt man pro Mol an 5-Chlor-1-alkyl-3-fluoralkyl-1H-pyrazol-4-carbaldehyd der Formel (II) im Allgemeinen zwischen 0,8 und 1,8 mol, bevorzugt zwischen 1 und 1,5 mol, an Fluorierungsmittel der Formel (III) ein.

Die Reaktion kann in Substanz oder in Gegenwart eines Lösungsmittels durchgeführt werden. Geeignete Lösungsmittel sind: Sulfolan, Dimethylsulphoxid (DMSO), Dimethylacetamid, Dimethylformamid (DMF), N-Methylpyrrolidon (NMP), 1,3-Dimethylimidazolinon, Dichlormethan, Chloroform, Dichlorethan. Nitrile, wie Acetonitril, Propionitril, n- oder i-Butyronitril oder Hexamethylphosphorsäuretriamid. Besonders bevorzugt verwendet man Sulfolan, DMSO, Dimethylacetamid, DMF oder NMP.

Die Fluorierung kann durch Zusatz von Phasen-Transfer-Katalysatoren beschleunigt werden.

Die bei der Durchführung des erfindungsgemäßen Verfahrens als Phasen-Transfer-Katalysatoren verwendbaren quartären Ammonium-, Phosphoniumverbindungen oder Amidophosphonium-Salze: wie Tetrabutylphosphoniumchlorid, Tetrabutylphosphoniumbromid, Tetraphenylphosphoniumchlorid oder Tetraphenylphosphoniumbromid, Tetrakis(dimethylamino)phosphoniumchlorid oder -bromid, Tetrakis(diethylamino)-phosphoniumchlorid oder -bromid, Tetrakis(dipropylamino)phosphoniumchlorid oder -bromid, Tris(di-ethylamino)(dimethylamino)phosphoniumchlorid oder -bromid, Tris(dimethylamino)(cyclohexylamino)-phosphoniumchlorid oder -bromid, Tris(dimethylamino)(diallylamino)phosphoniumchlorid oder -bromid, Tris(dimethylamino)(dihexylamino)phosphoniumchlorid oder -bromid, Tris(diethylamino)(dihexylamino)-phosphoniumchlorid oder -bromid, Tris(pyrrolidino)(ethylmethylamino)phosphoniumchlorid oder -bromid, Tris(pyrrolidino)(diethylamino)phosphoniumchlorid oder -bromid, Hexaalkyguanidiniusalze oder Polyethylenglykoldimethylethem der Kettenlängen r von 6 bis 17 und einer mittleren Molmasse von 500 g/mol.

### Schritt 2: Chlorierung

Die als Ausgangsstoffe benötigten 5-Fluor-1-alkyl-3-fluoralkyl-1H-pyrazol-4-carbaldehyde der Formel (IV) sind teilweise neu (z.B. 5-Fluor-1-alkyl-3-trifluormethyl-1H-pyrazol-4-carbaldehyde ist in WO 2004/014138 beschrieben).

Rest R¹ steht in dieser Formel (IV) bevorzugt für Methyl, Ethyl, n-Propyl, Isopropyl, Butyl, Pentyl, besonders bevorzugt für Methyl. Der Rest R² steht für C₁-C₅-Fluoralkyl, worin Fluoralkyl eine Alkylgruppe mit 1 bis 5 Kohlenstoffatomen bedeutet, welche mit mindestens einem Fluoratom bis hin zur Perfluorierung substituiert ist. Wenn das Fluoralkyl nicht perfluoriert ist, können weitere Halogenatome wie Chlor und Brom, bevorzugt Chlor, als weitere Substituenten vorhanden sein. R² steht bevorzugt für CF₂H, CF₃, CF₂Cl, CCl₂F, C₂F₅, C₃F₇, besonders bevorzugt für CF₂H und CF₃. Ganz besonders bevorzugt sind 5-Fluor-1-methyl-3-difluormethyl-1H-pyrazol-4-carbaldehyd (IV-1) und 5-Fluor-1-methyl-3-trifluormethyl-1H-pyrazol-4-carbaldehyd (IV-2), insbesondere die Verbindung (IV-1).

Die Chlorierung von Pyrazolaldehyden zum Säurechloriden wurde in WO 2008/086962 beschrieben.

Man setzt üblicherweise den 5-Fluor-1-alkyl-3-fluoralkyl-1H-pyrazol-4-carbaldehyd der Formel (IV) und das Chlorierungsmittel im Molverhältnis 1 : 3 bis 1 : 2, bevorzugt 1 : 1,4 bis 1 : 1 ein.

Als Chlorierungsmittel kann man Chlor, oder ein Chlor-abgebendes Agens einsetzen. Die Reaktion kann gegebenenfalls in Gegenwart eines inerten Verdünnungsgases wie z.B. Stickstoff, Kohlendioxyd oder Edelgase durchgeführt werden. Geeignet als Chlorierungsmittel, ohne Anspruch auf Vollständigkeit zu erheben, sind beispielsweise Cl₂, SO₂Cl₂, SOCl₂, N-Chlorsuccinimid oder ein Gemisch derselben. Man setzt bevorzugt Cl₂, SO₂Cl₂, oder ein Gemisch derselben als Chlorierungsmittel ein. Besonders bevorzugt ist SO₂Cl₂ und Cl₂ als Chlorierungsmittel.

Die Umsetzung von 5-Fluor-1-alkyl-3-fluoralkyl-1H-pyrazol-4-carbaldehyden der Formel (IV) mit dem Chlorierungsmittel erfolgt üblicherweise in Gegenwart eines Verdünnungsmittels, das sich unter den vorherrschenden Reaktionsbedingungen inert verhält. Man kann als Verdünnungsmittel beispielsweise einfach oder mehrfach chlorierte aliphatische oder aromatische Kohlenwasserstoffe oder Gemische derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, 1,4-Dichlorbenzol, 1,2,3-Trichlorbenzol, 1,2,4-Trichlorbenzol, 4-Chlor-trifluormethylbenzol, 1,3,5-Trichlorbenzol, 2-Chlortoluol, 3-Chlortoluol, 4-Chlortoluol oder ein Gemisch derselben. Besonders bevorzugt verwendet man Chlorbenzol und Dichlorbenzol.

Gemäß der vorliegenden Erfindung erfolgt die Chlorierung unter radikalischen Bedingungen. Voraussetzung dafür ist die Bildung von Chlorradikalen.

Es ist bekannt, dass organische Peroxide oder Azoverbindungen unter der Einwirkung von Wärme und/oder Licht in Radikale zerfallen, die die radikalische Chlorierung initiieren.

Beispiele, ohne Anspruch auf Vollständigkeit, geeigneter Peroxide und Azoverbindungen sind tert.-Butylhydroperoxyd, Dibenzoylperoxid, Di(4-tert-butylcyclohexyl)peroxydicarbonat, 2,2'-Azobis(isobutyronitril), Dimethyl-2,2'-azobis(isobutyrat), 2,2'-Azobis(2,4-dimethylvaleronitril), Di-(2-ethylhexyl)peroxydicarbonat, tert-Butylperoxypivalat, tert.-Amylperoxy-2-ethylhexanoat, tert-Butylperoxy-2-ethylhexanoat.

Bevorzugt setzt man folgende Radikalstarter ein: 2,2'-Azobis(isobutyronitril), 2,2'-Azobis(2,4-dimethylvaleronitril), Di(2-ethylhexyl)peroxydicarbonat, tert-Amylperoxy-2-ethylhexanoat, tert-Butyl peroxy-2-ethylhexanoat. Man setzt den Radikalstarter üblicherweise in einer Menge von 0,01 bis 1 Mol-%, vorzugsweise 0,1 bis 0,5 Mol-% bezogen auf den Aldehyd der Formel (IV) ein.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Anstelle der Chlorierung können die 5-Fluor-1-alkyl-3-fluoralkyl-1H-pyrazol-4-carbaldehyde der Formel (IV) alternativ durch Umsetzung mit einer Perhalosäure (z.B. Periodsäure) in Gegenwart eines Verdünnungsmittels (z.B. Acetonitril) und in Gegenwart eines Oxidationsmittels (z.B. PCC) in die 5-Fluor-1-alkyl-3-fluoralkyl-1H-pyrazol-4-carbonsäuren der Formel (V) 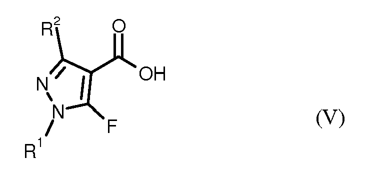 in welcher R¹ und R² die oben angegebenen Bedeutungen haben, umgesetzt werden.

Sowohl die 5-Fluor-1-alkyl-3-fluoralkyl-1H-pyrazol-4-carbaldehyde der Formel (IV) als auch die 5-Fluor-1-alkyl-3-fluoralkyl-1H-pyrazol-4-carbonsäuren der Formel (V) sind wichtige Zwischenstufen in der Synthese von Pflanzenschutzmitteln (vgl. z.B. Europäische Patentanmeldung Nr. 09356038.7 und WO 2007/087906).

Das erfindungsgemäße Herstellen von 5-Fluor-1,3-dialkyl-1H-pyrazol-4-carbonsäurefluoriden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

### Beispiel 1: 5-Fluor-1-methyl-3-difluormethyl-1H-pyrazol-4-carbaldehyd (IV-1)

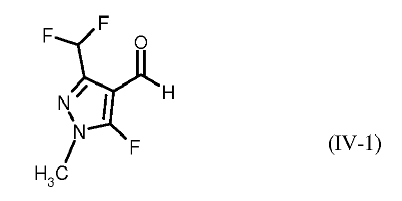

Unter Argon wurden 19,4 g (100 mmol) an 5-Chlor-1-methyl-3-difluormethyl-1H-pyrazol-4-carbaldehyd in Dimethylformamid vorgelegt. Anschließend wurden 8,6 g (150 mmol) Kaliumfluorid (Wassergehalt KF 0.05 %) zugegeben, auf 150°C erhitzt und bei dieser Temperatur für 8 Stunden nachgerührt. Anschließend wurde das Gemisch mit Ethylacetat verdünnt, filtriert und das Lösungsmittel in Vakuum bei 0,5 mbar und 70°C abdestilliert. Man erhält 16 g (90 % der Theorie) an 5-Fluor-1-methyl-3-difluormethyl-1H-pyrazol-4-carbaldehyd mit der Reinheit von 99 % (Schmelzpunkt 68°C).
¹H-NMR (CD₃CN): δ = 9.8 (1H, s), 6,88 (1H, t), 3,7 (3H, s ) ppm.
¹⁹F-NMR (CD₃CN): δ = -114,75 (2F, t), -124,06 (1F,s) ppm.

### Beispiel 1: 5-Fluor-1-methyl-3-difluormethyl-1H-pyrazol-4-carbaldehyd (IV-1)

96,3 g (1660 mmol) sprühgetrocknetes Kaliumfluorid wurden vorsichtig unter hohem Vakuum getrocknet. 129,2 g (664 mmol) 5-Chlor-1-methyl-3-difluormethyl-1H-pyrazol-4-carbaldehyd (II-1) in 1 L DMF wurden zugegeben. Unter schnellem Rühren wurde auf 150°C erhitzt. Die Reaktion ist nach 3 Stunden (GC-Kontrolle) abgeschlossen. Man kühlt auf Raumtemperatur und gibt Wasser bis zum einem Gesamtvolumen von 4 L zu. Das Reaktionsgemisch wird zweimal mit je 2000 ml Ethylacetat gewaschen. Die vereinigten organischen Phasen wurden anschließend mit gesättigter Natriumchloridlösung gewaschen, über Natriumsulfat getrocknet und eingeengt. Man erhält 117,6 g (90 % der Theorie, Reinheit 90 %) an 5-Fluor-1-methyl-3-difluormethyl-1H-pyrazol-4-carbaldehyd (IV-1) in Form eines braunen Feststoffes, welcher ohne weitere Aufreinigung weiter umgesetzt wird.

### Beispiel 3: 5-Fluor-1-methyl-3-difluormethyl-1H-pyrazol-4-carbonsäurechlorid (I-1)

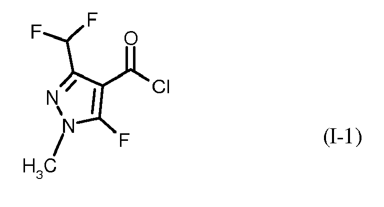

Die Lösung von 17.8 g (100 mmol) von 5-Fluor-1-methyl-3-difluormethyl-1H-pyrazol-4-carbaldehyd, 17,5 g (130 mmol) Sulfurylchlorid und 0,2 g 2,2-Azoisobutyronitril in 50 ml Chlorbenzol wurde bei 80°C für 6 Stunden gerührt. Die Reaktionslösung wurde eingeengt.

Man erhielt 20.1 g des Produktes als Öl mit der Reinheit (GC) von 98 %.
¹H-NMR (CD₃CN): δ = 6,88 (1H, t), 3,7 (3H, s ) ppm.

### Beispiel 4: 3-(Difluormethyl)-5-fluor-1-methyl-1H-pyrazol-4-carbonsäure (V-1)

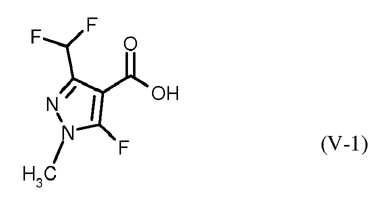

Eine Suspension von 79,7 g (350 mmol) Periodsäure in 640 ml absolutem Acetonitril wurde für 30 min gerührt. Bei 0°C wurden 56,6 g (318 mmol) 5-Fluor-1-methyl-3-difluonnethyl-1H-pyrazol-4-carbaldehyd (IV-1) und 1,4 g (6 mmol) PCC, gelöst in 130 ml trockenem Acetonitril zugegeben. Das Reaktionsgemisch wurde anschließend bei Raumtemperatur für 2,5 Stunden gerührt. Nach Zugabe von 1600 ml Ethylacetat wurde nacheinander mit gesättigter Natriumchloridlösung/Wasser (1:1), gesättigter Natriummetabisulfitlösung und gesättigter Natriumchloridlösung gewaschen. Die organische Phase wurde über Natriumsulfat getrocknet und eingeengt. Man erhält 51,4 g (83 % der Theorie) an 3-(Difluormethyl)-5-fluor-1-methyl-1H-pyrazol-4-carbonsäure (V-1) als gelben Feststoff.

### Beispiel 5: Ethyl-4,4-difluor-3-oxobutanoat (VII-1)

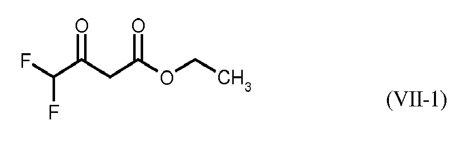

Unter Stickstoffatmosphäre wurden 46,7 g (1,168 mol) Natriumhydrid (60 % Dispersion in Parafin) zu 600 ml Tetrahydrofuran gegeben. Bei 35°C wurde ein Gemisch aus 125 g (1,008 mol) Difluorethylacetat und 88,7 g (1,010 mol) Ethylacetat zugetropft, wobei die Temperatur unter 40°C gehalten wurde. Anschließend wurde bei Raumtemperatur über Nacht weiter gerührt. Das Reaktionsgemisch wurde vorsichtig in 1,7 L Eiswasser geschüttet und der pH-Wert durch Zugabe von Schwefelsäure auf pH 3 eingestellt. Man extrahierte zweimal mit je 500 ml Methyl-tert-butyl-ether, wusch die vereinigten organischen Phasen zweimal mit gesättigter Natriumchloridlösung, trocknete über Natriumsulfat, engte bei 40°C und 150 mbar ein und destillierte bei 60 mbar (Vigreux-Kolonne). Das Produkt wurde bei 85-87°C als farblose Flüssigkeit erhalten (104 g, 62 % der Theorie mit einer Reinheit von > 99 % (GC)).

### Beispiel 6: 3-(Difluormethyl)-1-methyl-1H-pyrazol-5-ol (IX-1)

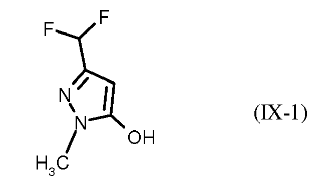

Zu einer Lösung von 208,6 g (1257 mmol) Ethyl-4,4-difluor-3-oxobutanoat (VII-1) in 1000 ml Toluol wurden unter Kühlung 57,8 g (1257 mmol) Methylhydrazin getropft. Nach 90 min unter Rückfluss wurde abgekühlt und eingeengt. Nach Zugabe von 1500 ml Diethylether wurde gerührt und unlösliches Material abfiltriert. Das Filtrat wurde eingeengt. Man erhielt 137,5 g (74 % der Theorie) an 3-(Difluormethyl)-1-methyl-1H-pyrazol-5-ol (IX-1) als rot-orangen Feststoff, welcher ohne weitere Reinigung im nächsten Reaktionsschritt eingesetzt wurde.

### Beispiel 7: 5-Chlor-3-(difluormethyl)-1-methyl-1H-pyrazol-4-carbaldehyd (II-1)

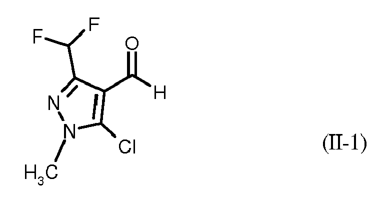

Zu einer Lösung aus 137,5 g (929 mmol) 3-(Difluormethyl)-1-methyl-1H-pyrazol-5-ol (IX-1) in 136 ml (1858 mmol) absolutem Dimethylformamid und 750 ml Toluol wurden unter Kühlung 571 g (3716 mmol) Phosphoroxychlorid zugetropft. Man erhitzte für 3 Stunden unter Rückfluss, ließ abkühlen und goss das Reaktionsgemisch vorsichtig in 4 L Eiswasser. Man extrahierte dreimal mit je 1500 mL Ethylacetat, wusch die vereinigten organischen Phasen zweimal mit gesättigter Natriumhydrogencarbonatlösung und schließlich mit gesättigter Natriumchloridlösung, trocknete über Natriumsulfat und engte ein. Man erhielt 129,2 g (72 % der Theorie mit einer Reinheit von > 99 % (GC)) an 5-Chlor-3-(difluormethyl)-1-methyl-1H-pyrazol-4-carbaldehyd (II-1) in Form eines rot-braunen Feststoffs, welcher ohne weitere Reinigung im nächsten Reaktionsschritt eingesetzt wurde.

## Patentansprüche

1. Verfahren zum Herstellen von 5-Fluor-1-alkyl-3-fluoralkyl-1H-pyrazol-4-carbonsäurechloride der Formel (I) 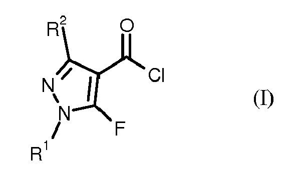 in welcher R¹ für C₁-C₆-Alkyl und R² für C₁-C₅-Fluoralkyl stehen, **dadurch gekennzeichnet, dass** man 5-Chlor-1-alkyl-3-fluoralkyl-1H-pyrazol-4-carbaldehyd der Formel (II) 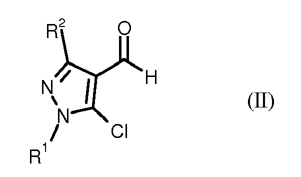 in welcher R¹ und R² die oben angegebenen Bedeutungen haben,
zuerst (Schritt 1) mit einem Fluorierungsmittel der Formel (III)
M⁺ F⁻ (III)
in welcher M⁺ für Li⁺, Na⁺, K⁺, Cs⁺, Alk₄N⁺ steht (worin Alk für C₁-C₄-Alkyl steht),
und gegebenenfalls in Gegenwart eines Phasen-Transfer-Katalysators zu 5-Fluor-1-alkyl-3-fluoralkyl-1H-pyrazol-4-carbaldehyd der Formel (IV) 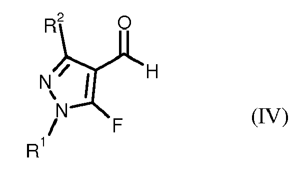 in welcher R¹ und R² die oben angegebenen Bedeutungen haben, umsetzt, und dann die Verbindungen der Formel (IV) durch Umsetzung mit einem Chlorierungsmittel (Schritt 2) in die Säurechloride der Formel (I) überführt.

2. Verfahren gemäß Anspruch 1, **dadurch gekennzeichnet, dass** man 5-Chlor-1-alkyl-3-fluoralkyl-1H-pyrazol-4-carbaldehyd der Formel (II) einsetzt, in welcher R¹ für Methyl, Ethyl, n-Propyl, Isopropyl, Butyl, Pentyl und R² für CF₂H, CF₃, CF₂Cl, CCl₂F, C₂F₅, C₃F₇ stehen.

3. Verfahren gemäß Anspruch 1, **dadurch gekennzeichnet, dass** man 5-Chlor-1-alkyl-3-fluoralkyl-1H-pyrazol-4-carbaldehyd der Formel (II) einsetzt, in welcher R¹ für Methyl und R² für CF₂H oder CF₃ stehen.

4. Verfahren gemäß Anspruch 1, **dadurch gekennzeichnet, dass** man 5-Chlor-1-methyl-3-difluormethyl-1H-pyrazol-4-carbaldehyd (II-1) einsetzt.

5. 5-Fluor-1-alkyl-3-fluoralkyl-1H-pyrazol-4-carbaldehyd der Formel (IV) 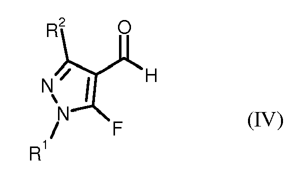 in welcher R¹ für C₁-C₆-Alkyl und R² für C₁-C₅-Fluoralkyl stehen.

6. 5-Fluor-1-alkyl-3-fluoralkyl-1H-pyrazol-4-carbaldehyd der Formel (IV) gemäß Anspruch 5, in welcher R¹ für Methyl, Ethyl, n-Propyl, Isopropyl, Butyl, Pentyl und R² für CF₂H, CF₃, CF₂Cl, CCl₂F, C₂F₅, C₃F₇ stehen.

7. 5-Fluor-1-alkyl-3-fluoralkyl-1H-pyrazol-4-carbaldehyd der Formel (IV) gemäß Anspruch 5, in welcher R¹ für Methyl und R² für CF₂H oder CF₃ stehen.

8. 5-Fluor-1-methyl-3-difluormethyl-1H-pyrazol-4-carbaldehyd (IV-1).

9. Verfahren zum Herstellen von 5-Fluor-1-alkyl-3-fluoralkyl-1H-pyrazol-4-carbaldehyd der Formel (IV) 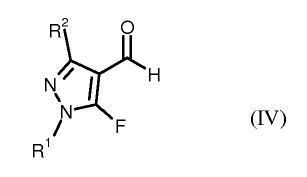 in welcher R¹ für C₁-C₆-Alkyl und R² für C₁-C₅-Fluoralkyl stehen,
**dadurch gekennzeichnet, dass** man 5-Chlor-1-alkyl-3-fluoralkyl-1H-pyrazol-4-carbaldehyd der Formel (II) 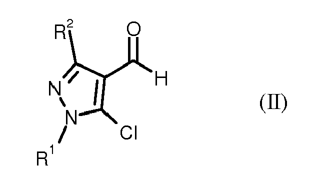 in welcher R¹ und R² die oben angegebenen Bedeutungen haben,
mit einem Fluorierungsmittel der Formel (III)
M⁺ F (III)
in welcher M⁺ für Li⁺, Na⁺, K⁺, Cs⁺, Alk₄N⁺ steht (worin Alk für C₁-C₄-Alkyl steht),
und gegebenenfalls in Gegenwart eines Phasen-Transfer-Katalysators umsetzt.

10. Verwendung von 5-Fluor-1-alkyl-3-fluoralkyl-1H-pyrazol-4-carbonsäurechloriden der Formel (I) gemäß Anspruch 1 oder von 5-Fluor-1-alkyl-3-fluoralkyl-1H-pyrazol-4-carbaldehyden der Formel (IV) gemäß Anspruch 5 zum Herstellen von Pflanzenschutzmitteln.
